(19) Europäisches Patentamt
European Patent Office
Office européen des brevets

(11) Publication number: **0 430 847 A1**

# (12) EUROPEAN PATENT APPLICATION

(21) Application number: **90500109.5**

(22) Date of filing: **28.11.90**

(51) Int. Cl.[5]: **C07C 211/52**, C07C 209/00, C07D 295/125

(30) Priority: **29.11.89 ES 8904064**

(43) Date of publication of application: **05.06.91 Bulletin 91/23**

(84) Designated Contracting States: **DE FR GB IT Bulletin**

(71) Applicant: **CENTRO MARGA PARA LA INVESTIGACION S.A.**
**Muntaner 212, 5o-510**
**E-08036 Barcelona(ES)**

(72) Inventor: **Nicolau, Claudio Palomo, Dr.**
**Aguirre Miram n, No 1, 3o-A,B, Barrio de Gros.**
**San Sebastián(ES)**
Inventor: **Castellvi, Francisco Cabré**
**Calle Casp 114, esc. B, 3o-1a.**
**E-08010, Barcelona(ES)**
Inventor: **Nicolau, Francisco Eugenio Palomo**
**Dos de Mayo, 34, 2o-1a**
**E-08190 Sant Cugat del Vallés, Barcelona(ES)**
Inventor: **Coll, Antonio Luis Palomo, Dr.**
**calle Escuelas Pias 18, 5o-1a**
**E-08017 Barcelona(ES)**

(74) Representative: **Ballester Rodés, Albert**
**Muntaner 212, 5o- 508**
**E-08036 Barcelona(ES)**

## (54) Procedure for the preparation of N-cyclopropyl-4-fluoroanilines.

(57) Procedure for the preparation of N-cyclopropyl-4-fluoroanilines of formula I. By selective reaction of II with cyclopropylamine, piperazines or pyrrolidines, III is obtained. Its acetylation and reduction yields IV which by deamination leads to I. $R^1$ is hydrogen, chlorine or methyl; $R^2$, hydrogen, halogen, methyl, a piperazinyl or a pyrroledinyl; $R^3$, hydrogen, halogen, hydroxyl or trifluoromethyl; and $R^4$, hydrogen, nitro, hydroxyl or amino.

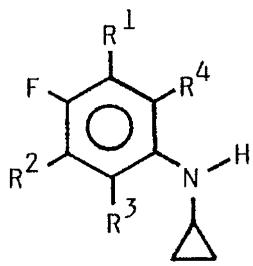
I

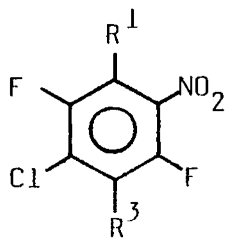
II

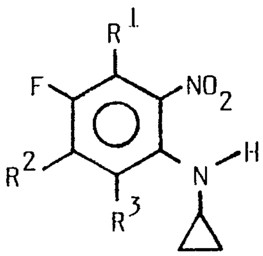
III

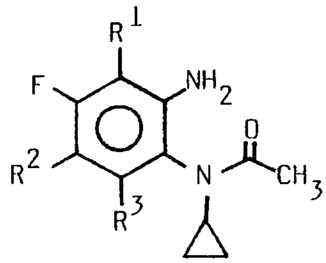
IV

The compounds of formula I are of interest on account of their application to the preparation of quinolone group antibiotics.

EP 0 430 847 A1

# PROCEDURE FOR THE PREPARATION OF N-CYCLOPROPYL-4-FLUOROANILINES

Description

The present invention is related to a procedure for the preparation of N-cyclopropyl-4-fluoroaniline derivatives which are intermediates of interest in the preparation of antibiotics of the quinolone group. Precisely, it includes a procedure for the preparation of piperazinyl, pyrroledinyl and pyrrolyl derivatives in the 3-position of N-cyclopropyl-4-fluoroaniline. Other derivatives are also the subject of this invention, particularly the N-cyclopropyl-3-chloro-4-fluoroaniline.

The intermediates of interest, prepared by the procedure described in this invention, are compounds represented by formula I,

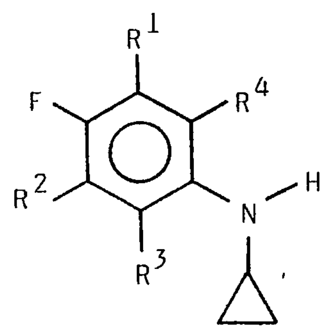

I

where substituents $R^1$, $R^2$, $R^3$ and $R^4$ represent: $R^1$, a hydrogen atom, chlorine or a methyl group; $R^2$, an atom of fluorine, chlorine, bromine, a methyl group, a piperazinyl, pyrrolyl, pyrrolidinyl or 2-oxo-1 ,3-oxazolidinyl; $R^3$, a hydrogen atom, chlorine, fluorine, hydroxyl or trifluorome thyl; and $R^4$, a hydrogen atom, a nitro group, hydroxyl or amino.

The procedure consists in the selective substitution reaction of 2-fluoro versus the 3-chloro by cyclopropylamine in nitrobenzenes of formula II,

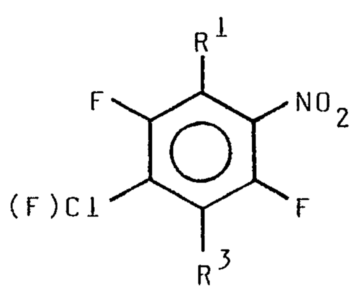

II

where $R^1$ and $R^3$ have the same meaning as before, to obtain another nitrobenzene of formula III,

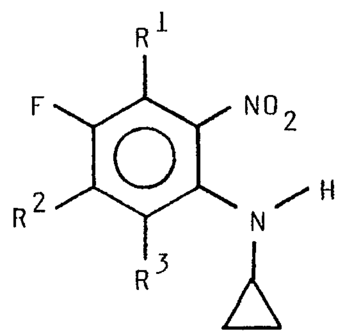

III

where $R^1$ and $R^3$ have the same meaning as before and $R^2$ an atom of chlorine or fluorine, which is next made to react with a piperazine, pyrrolidine or a nitrogen compound to obtain a compound or formula III, $R^1$, $R^2$ and $R^3$ having the same meaning as indicated before, provide $R^2$ is an atom of fluorine or chlorine.

The N-cyclopropylnitrobenzene is acetylated, yielding a compound of formula IV,

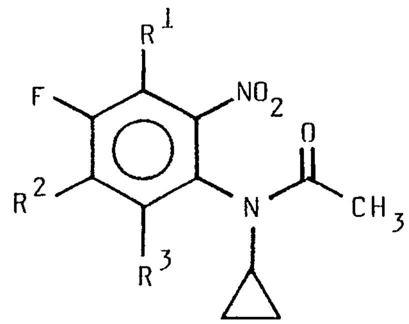

IV

the meaning of $R^1$, $R^2$ and $R^3$ being that given before. Reduction leads to compounds of formula V,

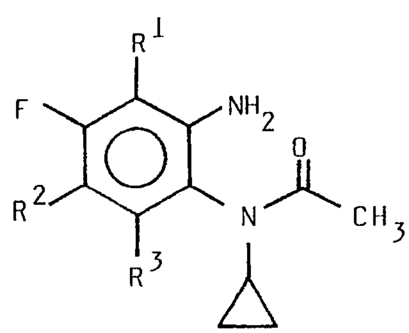

V

the meaning of $R^1$, $R^2$ and $R^3$ being that given before, which by acidic hydrolysis give compounds of formula I, $R^4$ being an amino group.

Reductive diazotation of the above-mentioned anilines, by means of alkaline nitrites and reductants, gives a compound of formula VI,

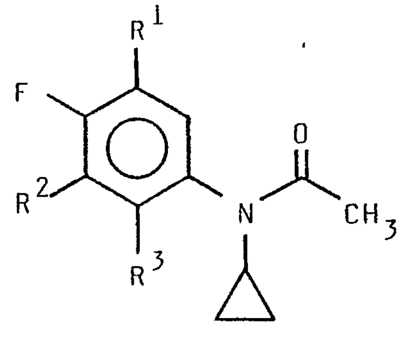

VI

the meaning of $R^1$, $R^2$ and $R^3$ being as before, which by hydrolysis yield a compound of formula I, being $R^4$ a hydrogen atom.

All the above-mentioned compounds may be isolated either as bases or as acid salts.

The preparation of the compounds N-cyclopropyl-3-chloro-4-fluoroaniline (Ia) and N-cyclopropyl-3-(4-$R^5$-piperazinyl-1)-4-fluoroaniline (Ib), where $R^5$ may represent a hydrogen atom, methyl, ethyl or cyclopropyl, involve the selective reaction of a nitrobenzene of formula II, being $R^1$ and $R^3$ hydrogen atoms, first with cyclopropylamine to obtain N-cyclopropyl-3-chloro-4-fluoro-6-nitroaniline (IIIa) and next with a piperazine to obtain N-cyclopropyl-3-(4-$R^5$-piperazinyl-1 )-4-fluoro-6-nitroaniline (IIIb), where $R^5$ has the meaning given above.

The N-acetyl derivatives IVa and IVb are prepared by acetylation of IIIa and IIIb. By reduction of these nitro compounds amines Va and Vb are obtained:

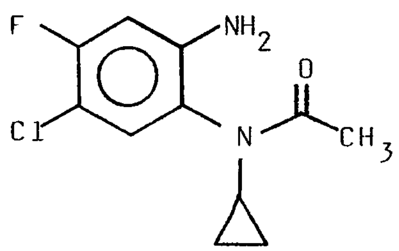

Va

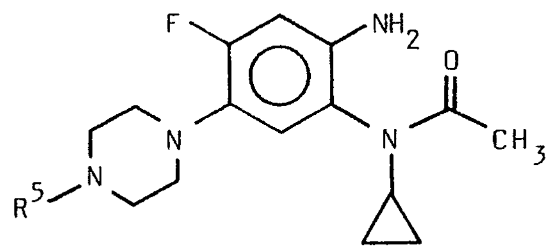

Vb

the meaning of $R^5$ being that already given. By reductive diazotation the de-aminized compounds VIa and VIb result which by hydrolysis, yield the fluoroanilines of formula Ia, being $R^1$ and $R^3$ hydrogen atoms and $R^2$ a chlorine atom, and Ib, being $R^1$ and $R^3$ hydrogen atoms and $R^2$ a piperazine.

The process of the present invention is characterized by the following experimental techniques:

1. Selective substitution of fluorine atom at the 2-position of the nitrobenzene of formula II by cyclopropylamine, this being conveniently accomplished in dimethylsulfoxide, dimethylformamide or a mixture of them, regulating the temperature from 20 to 50 oC. In this manner, the preparation of the compounds III, being $R^2$ a chlorine or fluorine atom, is achieved. Next, and without requiring the isolation of III, the substitution of $R^2$, this being a halogen, is effected with piperazine, 3-methylpiperazine, 4-acetyl-3-methylpiperazine, 4-ethylpiperazine, 4-methylpiperazine, 4-cyclopropylpiperazine, pyrrole, 3-aminoethylpyrrolidine, 3-aminomethylpyrrolidine, 3-aminopyrrolidine, 3-acetylaminopyrrolidine, 3-hydroxypyrrolidine, 3-hydroxymethylpyrrolidine, 3-cyclopropylaminopyrrolidine and 2-oxo-1,3-oxazolidine.

2. Acetylation with acetic anhydride or acetyl chloride. In the first case, the acetylation is catalyzed with a strong acid such as sulfuric acid, methanesulfonic acid or trichloroacetic acid. The acetylation may also be effected selectively, first at the piperazino group versus the cyclopropylamino of compounds III, being $R^2$ a 1-piperazinyl, aminopyrrolidino, alkylaminopyrrolidino or hydroxypyrrolidino, which may be substituted by C1-C3 alkyls. This is advantageous as far as assisting purification.

3. Reduction of the nitro group, which is performed with sodium borohydride and three equivalents of a nickel salt such as nitrate, sulfate, acetate and chloride, at a temperature from -5 to 25o C. Either 105

palladium-active carbon, at a temperature from 5 to 25 oC and pressure from 1.0 to 2.0 bar, or sodium borohydride-chlorotrimethylsilane at low temperature are also suitable.

Other reductive methods, such as aluminum-hydrochloric acid, stannous chloride-hydrochloric acid, sodium sulfide and hydrazine-iron catalyst, have failed.

Those amino compounds oxidize readily, giving strongly colored compounds. Because of it methods where the reduction cannot be controlled at temperatures near 0-5 oC have failed.

4. Reductive diazotation for the de-amination, a technique which, in general, takes place in low yields and the formation of resinous products occurring mainly by effect of temperature with those anilines. The method described in this invention is characterized because the reduction takes place at moderate temperatures. The diazotation is performed preferentially with sodium nitrite in an aqueous medium, and the reduction, by means hypophosphorous acid at room temperature in the presence of a copper compound selected among nitrate, sulfate, oxide, chloride, phosphate, hypophosphite and phosphite.

The hydrolysis for the de-acetylation is carried out in an acidic medium.

The selective reaction with compounds of formula II can be effected by employing first primary amines, such as ethylamine, isopropylamine, 2-chloroethylamine, 2-bromoethylamine, 2-hydroxyethylamine, and next proceeding to the substitution reaction at the 3-position with cyclopropylamine. Alternatively, products III, being $R^2$ a chlorine atom, could be made to react with a substituted pyrroline, pyrrolidine or imidazoline.

Some compounds described in the present invention have not been described before.

All melting points have been determined in Gallenkamp apparatus and are uncorrected. The boiling points have been determined by the oven temperature of a Kugelröhr, model Buchi GKR-50. The H-NMR have been recorded by means a Varian EM 360 A, 60 MHz spectrometer. The thin layer chromatografies have been performed on Merck, Kiesegek 60F-254 chromatographic plates.

Example 1

2,5-Difluorochlorobenzene

48 mL (23.5 cmole) of tetrafluoroboric acid (35% by volume) are diluted with 40 mL of water. To the resulting colorless solution 14.60 g (10.0 cmole) of 3-chloro-4-fluoroaniline are added, resulting a light-brown solution. It is cooled down to 4-6 oC, and at 6 oC the formation of a white crystalline product is observed. A solution of 6.92 g (10.0 cmole) of sodium nitrite in 14 mL of water is added gradually into the resulting suspension, causing re-dissolution followed by precipitation of a light-brown crystalline compound. That addition is effected in 10-15 min., keeping the temperature at 3-5 oC by means of an ice-water bath. At the end of the addition a suspension results which is stirred during 15 min. The precipitate is filtered off and washed successively with 16 mL of tetrafluoroboric acid, 5%, 20 mL of cool methanol and 20 mL of ethyl ether. A slightly yellow-tinted amorphous solid is obtained, which after drying, consists in 22.34 9 (91.44%) of the salt 3-chloro-4-fluorobenzenediazonium tetrafluoroborate, decomposing at 142-144 oC.

The preceding tetrafluoroborate salt is heated slowly in an oil bath kept at about 160 oC. The decomposition of the product results in a red liquid which can easily be stirred. After about 5 min. (with the same external temperature) that liquid thickens and white fumes of boron trifluoride are evolved, and a yellow liquid starts to distill. Next, the external temperature is lowered to 145-150 oC, letting the distillation to proceed until a small proportion of liquid is left behind, the temperature is raised again up to 165-170oC until the distillation is over. The yellowish distillate is re-distilled at atmospheric pressure yielding 10.49 g (70.6%) of a colorless liquid boiling at 129-131 °C which corresponds to the title compound, its purity being adequate to be used in the following stage.

Characteristics of the product:

Mol. weight = 148.537 g/mole.

Mol. weight (tetrafluoroborate salt) = 244.360 g/mol.

bp (760 mm.Hg) = 129-131 °C.

dec. (tetrafluoroborate salt) = 142-144 °C.

TLC (n-hexane as the eluent), a sole spot Rf = 0.8-0.9.

$^1$H-RMN($CDCl_3$, δ ppm): 7.33-6.60 (m,arom.).

By substituting in this example the aniline by an equivalent amount of 3-chloro-2,4-difluoroaniline, 2,3,6-trifluorochlorobenzene results in a similar yield. The 2-chloro-3,6-difluorochlorobenzene is obtained in the same manner starting from the corresponding aniline.

Example 2

4-Chloro-2 ,5-difluoronitrobenzene

30.6 mL of conc. $H_2SO_4$ (98% by weight) are cooled down to 5-10 oC. Next 14.85 g (10.0 cmole) of 2,5-difluorobenzene are gradually added, and the resulting colorless emulsion is cooled down to 5-0 oC. Then 8.14 mL (10.69 cmole) of conc. $HNO_3$ (60% by weight) is gradually added during 60 min., keeping the temperature of the system under 20 oC. At the end of the reaction, a yellow crystalline precipitate is observed.

The mixture is left at that temperature during 60 min. with stirring. The resulting yellow suspension is slowly added into 100 mL of water previously cooled at 0-5 oC, giving an orange-red emulsion which is extracted twice with 100 mL of $CH_2Cl_2$. The organic layer is washed with 100 mL of water, 50 mL of NaOH 1.0 N and 50 mL of water, it is dried over anh. $Na_2SO_4$, and evaporated under reduced pressure. 18.67 9 (96.7%) of a yellow liquid are obtained which is submitted to distillation giving 16.48 g (85.18%) of a light-yellow distillate boiling at 112-114 oC (16 mm Hg). This liquid crystallizes yielding the title compound, yellow color, mp 27-29 oC.

Characteristics of the product:
Mol. weight = 193.535 g/mole.
mp = 27-29 °C (distilled product).
TLC (n-hexane as the eluent), a sole spot Rf = 0.5.
$^1$H-RMN ($CDCl_3$, δ ppm): 7.70 (1H,dd,$J_{H-F(ortho)}$ = 10 Hz,$j_{H-F(meta)}$ = 6 Hz), 7.33 (1H,dd,$j_{H-F(ortho)}$ = 10 Hz,$j_{H-F-(meta)}$ = 6 Hz).
$^1$H-RMN (300 MHz, $CDCl_3$, δ ppm): 7.941 (1H,dd,arom.,$j_{H-F(ortho)}$ - 7.95 Hz,$j_{H-F(meta)}$ = 6.46 Hz), 7.442 (1H,dd, arom., $j_{H-F(ortho)}$ = 9.77 Hz,$j_{H-F(meta)}$ = 5.86 Hz).
IR (in $CH_2Cl_2$, $cm^{-1}$): 1598 (N-O, ass. str.), 1341 (N-O, sim. str.).
Following the reaction:

The product obtained is compared chromatographically with the starting material, eluting with n-hexane; the nitrated compound displays a lower Rf (Rf = 0.5) than the starting material (Rf = 0.8-0.9).

Employing the same nitration procedure, 2,3,6-trifluoro-4-nitrochlorobenzene, 2-chloro-3,6-difluoro-4-nitrobenzene and 2-methyl-3,6-difluoro-4-chloronitrobenzene are obtained in similar yields.

Example 3

N-Cyclopropyl-3-chloro-4-fluoro-6-nitroaniline

To a yellow solution of 19.35 g (10.0 cmole) of 4-chloro-2,5-difluoronitrobenzene in 100 mL of dimethylsulfoxide 10.60 g (10.0 cmole) of anh. sodium carbonate are added, the color changing to orange-red. The resulting suspension is cooled down to 15 oC and next 8.56 g (15.0 cmole) of cyclopropylamine are slowly added, controlling the exothermicity of the reaction by means of an ice-water bath so that the temperature does not exceed 20-30 oC. At the end of the addition a color change to reddish orange is observed. The mass is kept under stirring for 60 min. at 25-30 oC, at the end of that time a reddish suspension being obtained. The resulting mixture is filtered, and most of the solvent is distilled off under reduced pressure. The residue is poured over 150 mL of water previously cooled at 0-5 oC, and a reddish oil separates, which is extracted twice with 100 mL of dichloromethane. The organic layer is washed successively with 100 mL of a saturated aqueous solution of NaCl, and 200 mL of water, dryed over anh. sodium sulfate, and evaporated under reduced pressure. 25.82 g of a reddish oil are obtained, which crystallizes; after washing it with 10 mL of n-hexane 12.03 g of an orange crystalline solid mp = 64-67 oC are obtained. From the mother liquors more product crystallizes out which is filtered and washed with 5 mL of n-hexane yielding 2.58 g of a crystalline solid of same melting point. 22.0 (95.0%) of the title compound result, being sufficiently pure to be used in next stage.

Characteristics of the product:
Mol. weight = 230.622 g/mole.
mp (crude) = 64-67 °C.
mp (recrystallized) = 73-75 °C (the product recrystallized from n-hexane; an orange crystalline solid).
TLC (n-hexane as the eluent), a sole spot Rf = 0.4.

[1]N-RMN ($CDC1_3$, δ ppm): 8.00-7.50 (1H,wide absorption,N-H), 7.73 (1H,d,arom.,$j_{H-F(ortho)}$=10 Hz), 7.17 (1H,d,arom., $j_{H-F(meta)}$=6 Hz), 2.60-2.13 (1H,m,CH), 1.17-0.23 (4H,m, $CH_2CH_2$).

$^1$H-RMN (300 MHz, $CDCl_3$, δ ppm): 7.977 (1H, wide absorption,NH), 7.972 (1H,d,arom.,$j_{H-F(ortho)}$=9.28 Hz), 7.378 (1H,d,arom.,$j_{H-F(meta)}$=6.35 Hz), 2.579-2.526 (1H,m,CH), 0.973-0.930 (2M,m,$CH_2$), 0.702-0.649 (2H,m,$CH_2$).

IR (KBr, $cm^{-1}$): 3364 (N-H), 1500 (N-0, ass. str.).

Microanalysis:

Calculated: C, 46.87, H, 3.50, N, 12.15%.

Found: C, 46.54, H, 3.41, N, 11 .69%.

Following the reaction:

By TLC (eluting with n-hexane); the reaction product displays an Rf = 0.4 slightly lower than that of the starting material (Rf = 0.5); in addition its chromatographic spot is orange colored.

Applying the same procedure N-cyclopropyl-2,3-dichloro-4-fluoro-6-nitroaniline and N-cyclopropyl-3-chloro-2,4-difluoro-6-nitroaniline result in similar yields.

Example 4

N-Cyclopropyl-4-fluoro-6-nitro-3-piperazinylaniline

23.06 9 (10.0 cmole) of N-cyclopropyl-3-chloro-4-fluoro-6-nitroaniline are added to 100 mL of dimethylsulfoxide. To the orange solution 21.53 9 (25.0 cmole) of anhydrous piperazine are added which remains partly insoluble. It is then heated to 90-95 oC, total dissolution being observed at 50-60 oC. The resulting intense red solution (the color is enhanced by heating) is maintained at 90-95 oC for 60 min. Next, it is cooled down to room temperature and added onto 200 mL of water previously cooled to 0-5 oC; the orange precipitate is filtered off through celite, washed with 100 mL of water, and the solid dissolved in 100 mL of methanol. The orange solution is evaporated at reduced pressure. The resulting solid residue is dissolved in 100 mL of dichloro methane and washed successively with 50 mL of a saturated solution of sodium chloride and with 50 mL of water. The organic layer is dried over anhydrous sodium sulfate and evaporated under reduced pressure giving a red oil which crystallizes under cooling; by washing with 20 mL of n-hexane it yields 21.27 g (75.9%) of an amorphous, orange solid of the title compound being of sufficient purity to be used in next stage. From the mother liquors an additional 13% of the product is recovered.

Characteristics of the product:

Mol. weight = 280.293 g/mole.

mp (recrystallized) = 107-110 °C (in ethanol).

TLC (ethyl acetate/n-hexane as the eluent), a sole spot Rf = 0.2.

$^1$H-RMN ($CDCl_3$, δ ppm): 8.43-8.06 (1H,wide absorption,N-H), 7.73 (1H,arom.,d,$j_{H-F(ortho)}$=14 Hz), 6.50 (1H,arom.,d, $j_{H-F(meta)}$=8 Hz), 3.60-2.56 (8H,m,$2CH_2CH_2$), 2.90-2.23 (1H, m,CH), 1.83 (1H,moderately wide absorption,NH), 1.00-0.30 (4H, m,$CH_2CH_2$).

$^1$H-RMN (300 MHz, $CDCl_3$, δ ppm): 8.247 (1H,wide absorption,NH), 7.850 (1H,d,arom.,$j_{H-F(ortho)}$=13.91 Hz), 6.552 (1H,d,arom.,$j_{H-F(meta)}$=7.76 Hz), 3.498-3.271 (4H, m,$CH_2CH_2$), 3.100-3.080 (4H,m,$CH_2CH_2$), 2.562-2.511 (1H,m,CH), 2.158 (1H,wide absorption,NH), 0.943-0.881 (2H,m,$CH_2$), 0.689-0.638 (2H,m,$CH_2$).

IR(KBr, $cm^{-1}$): 3330, 3220 (N-H), 1500(N-O, ass. str.).

Microanalysis:

Calculated: C, 55.71, H, 6.11, N, 19.99%.

Found: C, 55.30, H, 6.08, N, 19.60%.

Following the reaction:

The Rf of the product when eluting with ethyl acetate/n-hexane (1/2) is 0.0 and that of the starting material, 0.8.

By substitution of the piperazine by N-acetyl-, N-methyl-, N-ethyl- or N-cyclopropylpiperazine the corresponding derivatives are obtained in a similar yield.

Following the same procedure and substituting the piperazine by 3-hydroxypyrrolidine, 3-hydroximethylpyrrolidine, 3-acetylaminopyrrolidine, and the 3-chloro derivative by N-cyclopropyl-2,3-dichloro-4-fluoro-6-nitroaniline or 3-chloro-2,4-difluoro-6-nitroaniline, the 3-chlorine-substitution derivatives are obtained.

Example 5

N-Cyclopropyl-4-fluoro-6-nitro-3-( 1-piperacinyl )aniline

To a solution of 19.35 9 (10.0 cmole) of 4-chloro-2,5-difluoronitrobenzene in 100 mL of dimethylsulfoxide 10.60 g (10.0 cmole) of anhydrous sodium carbonate are added, a color change from pale yellow to orange being observed. The resulting suspension is cooled down to 15 oC and 13.86 mL (20.0 cmole) of cyclopropylamine are slowly added, preventing the reaction temperature from raising above 30 oC; when the addition is complete the color changes to reddish-orange. Room temperature is kept for 75 min. after which a reddish suspension is obtained.

Next, 21.53 g (25.0 cmole) of anhydrous piperazine are added, which remains partly insoluble. By heating up to 85-90 oC a red solution is obtained which is kept within this temperature range for 30 min. with stirring. Then it is cooled down to room temperature and 400 mL of water at 0-5 oC are slowly added, the separation of a reddish dough taking place; this is extracted with 200 mL of dichloromethane, and the resulting organic layer is washed successively with 100 mL of a saturated aqueous solution of sodium chloride and with 200 mL of water. The organic layer is dried over anhydrous sodium sulfate and its solvent evaporated under reduced pressure. An orange oil results which solidifies with 40 mL of n-hexane, yielding 22.37 g (79.81%) of an orange, amorphous solid consisting in the title compound, being sufficient pure to be used in next stage. The filtration mother liquors yield 2.32 g more of product; the total amount is 24.69 g (88.08%).

Following the reaction:

In the substitution by cyclopropylamine, the reaction may be monitored by TLC (n-hexane as the eluent) being 0.4 the Rf of the product, which is lower than that of the initial product, 0.5. In the substitution by piperazine the reaction may be followed by TLC (ethyl acetate/n-hexane 1/2 as the eluent), being 0.0 the Rf of the final product, and 0.8 that of the monosubstitution product (N-cyclopropyl-3-chloro-4-fluoro-6-nitroaniline).

Example 6

N-Cyclopropyl-4-fluoro-6-nitro-3-(N-acetyl-1-piperazinyl)-acetanilide

Under stirring, to 100 mL of acetic anhydride, 32.23 g (10.0 cmole) of N-cyclopropyl-4-fluoro-6-nitro-3-(N-acetylpiperazinyl)-aniline are added, causing a slight exothermicity. To the resulting orange suspension 40 drops of conc. sulfuric acid (98% by volume) are added (slight exothermicity). Then, it is heated up to 70-75 oC, a solution being formed at 50-55 oC, which is kept at the same temperature for 60 mitt., the reddish color turning darker. Next, the mass is cooled down to 0-5 oC, and 66 mL of methanol are gradually added, keeping the reaction temperature around 20 oC for 60 min. Next, it is left to reach room temperature, and the resulting reddish solution is let to stand for 30 mitt. at this temperature. After cooling down to 0-5 oC, 330 mL of an aqueous saturated solution of sodium bicarbonate and 66 mL of 2.0 N sodium hydroxide are gradually added; the mass is extracted twice with 100 mL of dichloromethane, and the organic layer is dried over anh. sodium sulfate, and its solvent is evaporated under reduced pressure. A light-brown solid is obtained which is washed with 100 mL of n-hexane, yielding 31.80 g (87.30%) of the title compound which is pure enough to be used in the following stage.

Characteristics of the product:

Mol. weight = 364.369 g/mole.

mp = 162-164 °C (from ethanol, light brown crystalline solid).

mp (second recrystallization) = 166-168 °C.

TLC ($CHCl_3$/methanol 9.5/0.5 as the eluent) a sole spot Rf = 0.8.

$^1$H-RMN ($CDCl_3$, $\delta$ ppm): 7.83 (1H,arom,d,$j_{H\text{-}F(ortho)}$ = 12 Hz), 6.50 (1H,arom,d,$j_{H\text{-}F(meta)}$ = 8 Hz), 4.17-2.80 (9H,m,two wide absorptions from $2CH_2CH_2$, and wide absorption due to CH), 2.33(90% 3H,s,$CH_3$ of an isomer), 2.10 ([10% 3H,s,$CH_3$ of another isomer],[3H,s,$CH_3$]), 1.17-0.40 (4H,m,$CH_2CH_2$).

$^1$H-RMN (300 MHz, $CDCl_3$, $\delta$ ppm.): 7.822 (1H,dd,$j_{H\text{-}F(ortho)}$ = 12.69 Hz), 6.559 (1H,arom,d,$j_{H\text{-}F(meta)}$ = 7.87 Hz), 3.836-3.649 (4H,wide absorption,$CH_2CH_2$), 3.320-3.097 (4H,wide absorption,$CH_2CH_2$), 3.169-3.110 (1H,m,CH), 2.393 (87.3% 3H,s, $CH_3$), 2.148 ([12.7% 3H,s,$CH_3$],[3H,s$CH_3$]), 0.949-0.683 (4H,wide absorption, $CH_2CH_2$).

IR(KBr, $cm^{-1}$): 1672(C=O), 1638 (C=O), 1519 (N-O ass. str.).
Microanalysis:
Calculated: C, 56.04, H, 5.81, N, 15.37%.
Found: C, 55.79, H, 5.87, N, 15.29%.
Following the reaction:

By TLC (ethyl acetate/n-hexane being the eluent); the Rf of the reaction product is 0.0-0.1, that of the starting material being Rf = 0.4.

Example 7

N-cyclopropyl-4-fluoro-6-nitro-3-(N-acetyl-1-piperazinyl)-aniline

28.03 g (10.0 cmole) of N-cyclopropyl-4-fluoro-6-nitro-3-piperazinylanaline are added to 150 mL of dichloromethane. The resulting orange solution is cooled down to 0-5 oC, and 20.79 mL (15.0 cmole) of triethylamine are added. Next, 11.0 mL (15.0 cmole) of acetyl chloride in 50 mL of dichloromethane are gradually added during 30 min., keeping the temperature between 5-10 oC, a crystalline precipitate being observed. The mass is let to reach room temperature and the resulting orange suspension is stirred during 30 min. Next it is washed successively with 100 mL of water, 100 mL of 0.1 N HCl, and 100 mL of a saturated aqueous solution of sodium bicarbonate; the organic layer is dried with anh. sodium sulfate, and the solvent is evaporated under reduced pressure giving an orange residue which, after washing it with 50 mL of n-hexane, yields 29.85 g (92.61%) of an amorphous red-brick solid consisting in the title compound, its purity being adequate to be used in next stage. The melting point of the raw product is 137-140 oC.

Characteristics of the product:
Mol. weight = 322.331 g/mole.
mp (crude) = 137-140 °C.
PF (recrystallized) = 139-141 °C (from ethanol, a red-brick crystalline solid).
TLC (ethyl acetate/n-hexane; 4/1 as the eluent) a sole spot Rf = 0.4.
$^1$H-RMN ($CDCl_3$, $\delta$ ppm): 8.23-7.97 (1H,wide absorption,N-H), 7.70 (1H,arom,d,$j_{H\text{-}F(ortho)}$ = 14 Hz), 6.43 (1H,arom,d, $J_{H\text{-}F(meta)}$ = 8Hz), 4.13-2.70 (8H,m(two wide absorptions), $2CH_2CH_2$), 2.60-2.20 (1H,m,CH), 2.03 (3H,s,$CH_3$), 0.97-0.23 (4H,m,$CH_2CH_2$).
$^1$H-RMN(300 MHz,$CDCl_3$, $\delta$ ppm): 8.244 (1H,wide absorption, NH), 7.847 (1H,arom,d,$j_{H\text{-}F(ortho)}$ =13.92 Hz), 6.549 (1H,arom, d,$j_{H\text{-}F(meta)}$=7.76 Hz), 3.811-3.661 (4H,2 separated wide absorptions, $CH_2CH_2$), 3.321-3.268 (4H,2 merged wide absorptions, $CH_2CH_2$), 2.537-2.519 (1H,m,CH), 2.156 (3H,s, $CH_3$), 0.941-0.879 (2,m,$CH_2$),0.686-0.652 (2H,m,$CH_2$).
IR (KBr, $cm^{-1}$): 3330 (N-H, wide absorption), 1640 (C=O), 1499 (N-O ass. str.).
Microanalysis:
Calculated: C, 55.89, H, 5.94, N, 17.38%.
Found: C, 55.04, H, 5.84, N, 17.03%.
Following the reaction:

By TLC (ethyl acetate/n-hexane 4/1 as the eluent); the product has an Rf = 0.4, that of the starting material being 0.2.

Employing the same procedure the 2-chloro and the 2-fluoro derivatives of the title compound are obtained in similar yields, starting from the respective anilines.

Example 8

N-Cyclopropyl-4-fluoro-6-nitro-3-(N-acetyl-1-piperazinyl)-acetanilide

To 100 mL of acetic anhydride at 15-20 oC 28.03 g (10.0 cmole) of 4-fluoro-6-nitro-3-piperazinyl-N-cyclopropylamine are gradually added (exothermic reaction). The resulting orange suspension is kept at room temperature during 30 mitt., and next 40 drops of conc. sulfuric acid (98% by weight) are added, causing some darkening.

The mass is heated up to 70 oC, and a dark-red solution is obtained which is kept at this temperature for 40 min. Next it is cooled down to 0-5 oC, and 66 mL of methanol are slowly added during 60 min., avoiding to exceed the temperature of 20 oC. The mass is it let to reach room temperature for 30 mitt. The

resulting solution is cooled down to 0-5 oC and next 330 mL of a saturated aqueous solution of sodium bicarbonate and 66 mL of 0.2 N sodium hydroxide are added; it is extracted twice with 100 mL of dichloromethene, and the organic layer is dried over anh. sodium sulfate, and evaporated to dryness under reduced pressure A dark-brown oil is obtained which solidifies upon addition of 25 mL of n-hexane; the solid is filtered and washed twice with 30 mL of n-hexane yielding 32.31 g (80.66%) of a yellow amorphous solid consisting in the title compound, provided with sufficient purity to be used in the next stage. mp = 150-155 °C. From the mother liquors an additional 10% of the product is recovered.

Following the reaction:

By TLC (ethyl acetate/n-hexane 4/1 as the eluent); the reaction product displays an Rf = 0.0-0.1, and that of the starting material, Rf = 0.2.

Following the same procedure, the acetyl derivatives of the anilines of example 5 result in a similar yield.

Example 9

N-Cyclopropyl-6-amino-4-fluoro-3-(N-acetyl-1-piperazinyl)-acetanilide

36.44 9 (10.0 cmole) of N-cyclopropyl-4-fluoro-6-nitro-3-(N-acetyl-1-piperazinyl)acetanilide are suspended in 375 mL of methanol. Next 47.50 g (20.0 cmole) of Ni(II) chloride hexahydrate are added, and the resulting greenish yellow suspension is cooled down to -10 oC. 15.10 g (40.0 cmole) of sodium borohydride are added in small portions onto this suspension, seeing that the temperature after each portion does not exceed 0-5 oC; after the first portion added darkening of the suspension is observed (finally, a black suspension is obtained), with strong exothermicity and hydrogen evolvement. The addition time is 60 min. approximately, the resulting black suspension being kept at 0-5 oC for 15-30 mitt.

Then 40 mL of water are slowly added, keeping the temperature within 0-5 oC, and next 200 mL of dichloromethane; 100 mL of 2.0 N hydrochloric acid are added and the mass filtered through celite to remove the black residue formed in the reaction. The aqueous layer is decanted and alkalinized with 5% sodium hydroxide, a light-brown oil being formed which is extracted twice with 100 mL of dichloromethane, the organic layer is dried over anh. sodium sulfate, and evaporated under reduced pressure, yielding 30.12 g (90.10%) of a light-brown amorphous solid consisting in the title compound, mp = 135-145 oC, of sufficient purity to be used in the next stage.

Characteristics of the product:

Mol. weight = 334.369 g/mole.

mp (crude) = 135-145 °C.

mp (purified) = 151-152 °C (white solid). TLC ($CHCl_3$/methanol; 9.5/0.5 as the eluent) a sole spot Rf = 0.8.

$^1$H-RMN ($CDCl_3$, $\delta$ ppm.): 6.83-6.27 (2H,m,arom,2CH), 3.97-2.67 (1H[8H,two wide absorptions,$2CH_2CH_2$], [1H,m,CH].[2H, wide absorption,$NH_2$]), 2.075 ([3H,s,$CH_3$],[28% 3H,s,$CH_3$]), 1.77 (72% 3H,s,$CH_3$ from isomer), 0.90-0.20 (4H,m,$CH_2CH_2$).

$^1$H-RMN (300 MHz, $CDCl_3$, $\delta$ ppm.): 6.528 (1H,d,arom,$j_{H-F(ortho)}$ = 13.0 Hz), 6.464 (1H,d,arom,$j_{H-F(meta)}$ = 8.54 Hz), 3.855-3.598 (6H[4H,m,$CH_2CH_2$],[2H,wide absorption, $NH_2$]), 3.394-3.318 (1H,m,CH), 2.972-2.857 (4H,m,$CH_2CH_2$), 2.133 ([3H,s, $CH_3$],[27%3H,s,$CH_3$]), 1.837 (73%3H,s,$CH_3$), 0.759-0.703 (2H,m,$CH_2$), 0.442-0.396 (2H,m,$CH_2$).

IR (KBr, $cm^{-1}$): 3480, 3400($NH_2$), 1670 (C=O), 1640 (C=O).

Following the reaction:

By TLC ($CHCl_3$/methanol 9.5/0.5 being the eluent); the reaction product has the same Rf (Rf = 0.8) as that of the starting material; nevertheless, its chromatographic spot absorbs only in the UV.

Applying the same method to the nitro compounds of example 4, acetylated previously according to examples 6 and 7, the corresponding 6-amino derivatives are obtained in similar yield.

Example 10

N-Cyclopropyl-4-fluoro-3-(N-acetyl-1-piperazinyl)acetanilide

A) With tert-butyl nitrite/DMF:

A solution of 33.44 g (10 cmole) of N-cyclopropyl-6-amino-4-fluoro-3-(N-acetylpiperazinyl)acetanilide in 108.64 mL of DMF is added dropwise to a solution of 21.24 mL (18.10 cmole) of tert-butyl nitrite in 16.34 mL of DMF at the temperature of 50-55 oC; the addition time is 20 min., and during the addition an evolvement of nitrogen is observed. The mass is stirred for 15 min. at the temperature of 50-55 oC, and then cooled down to room temperature. To the resulting brown solution 200 mL of dichloromethane are added, and it is washed twice with 500 mL of water. 20.63 g (64.6%) of a reddish oil are obtained which through silica is eluted with a mixture of ethyl acetate/n-hexane (1/1), yielding 12.04 g (37.71%) of a light-brown oil of the title compound being its purity sufficient to be used in the next stage.

B) With hypophosphorous acid-sodium nitrite:

33.4 g (0.10 mole) of N-cyclopropyl-6-amino-4-fluoro-3-(N-acetylpiperazinyl)acetanilide are added to a mixture of 50% hypophosphorous acid (164.0 mL, 1.50 mole), 96% sulfuric acid (0.20 mole) and copper sulfate (1.64 g, 0.01 mole) at room temperature. The resulting solution is cooled down to 0-5 oC and a solution of sodium nitrite (7.2 g, 0.104 mole) in water (50 mL) is added dropwise during 30-45 min. keeping the temperature between 0 and 5 oC. Since the start of the addition an evolvement of nitrogen, as well as a darkening of the solution (wine-red color), is observed. The reaction is left undisturbed for 90 min. at 10-15 oC, the color and the nitrogen evolvement fading gradually. When the reaction time is over, the mass is extracted three times with dichloromethane (200 mL each extraction), the organic layer is washed with an equal volume of 0.1 N sodium hydroxide, dried with anh. sodium sulfate and evaporated, yielding 27.1 g (0.085 mole, 85%) of an orange oil.

Characteristics of the product:

Mol. weight = 319.355 g/mole.

mp = an oil (from column chromatography).

TLC ($CHCl_3$/methanol; 9.5/0.5 as the eluent) a sole spot Rf = 0.9.

$^1$H-RMN ($CDCl_3$, $\delta$ ppm.): 7.40-6.50 (3H,arom.,3CH), 2.80 (9H,[8H,m,$2CH_2CH_2$],[1H,m,CH]), 2.10 (6H,s,$2CH_3$), 1.17-0.27 (4H,m,$CH_2CH_2$).

$^1$H-RMN (300 MHz, $CDCl_3$, $\delta$ ppm.): 7.051 (1H,dd,$j_{H\text{-}F(orto)}$ = 11.66 Hz, $j_{H\text{-}H}$=9.0 Hz), 6.680-6.520 (2H,arom, m,2CH), 3.798-3.764 (2H,m,$CH_2$), 3.645-3.613 (2H,m,$CH_2$), 3.105-3.041 (5H[4H,m,$2CH_2$],-[1H,m,CH]), 2.137(6H,s, $2CH_3$), 0.871-0.486 (4H,2 wide absorptions $CH_2CH_2$).

$^{13}$C-RMN ($CDCl_3$, $\delta$ ppm.): 169.059 (2 C=O), 122.089 (C-N(arom.)), 121.948 (C-N(arom.)), 118.550 (CH-(arom)), 116.683 (CH(arom.)), 116.413 (CH(arom.)), 108.495 (C-N), 50.623, 50.116 ($4CH_2$), 46.310, 41.359 ($2CH_2$)), 23.369, 21.329 ($2CH_3$).

IR ($CH_2Cl_2$, $cm^{-1}$): 1670 (C=O), 1640 (C=O).

Following the reaction:

By TLC ($CHCl_3$/methanol 9.5/0.5 as the eluent), the product having an Rf higher (Rf = 0.9) than that of the starting material (Rf = 0.8).

Example 11

N-Cyclopropyl-4-fluoro-3-(1-piperazinyl )aniline

31.9 g of N-cyclopropyl-4-fluoro-3-(4-acetyl-1-piperazinyl)-acetanilide (10.0 cmole) are added to a 25% aq. HCl solution (76.3 mL, 60.1 cmole) in methanol (53 mL). The resulting dark-orange solution is refluxed (74-76 oC) for 2 hours, lightening of the mixture being observed, a light-orange solution resulting at the end of the hydrolysis. The mixture is cooled down to 0-5 oC, and 80 mL of aqueous ammonia at 20 oC and dichloromethane (150 mL) are gradually added. After completing the addition, the organic layer is decanted and extracted with 150 mL of dichloromethane. After drying over anh. sodium sulfate and evaporation of the dichloromethane under reduced pressure, 22.1 g (9.4 cmole) of the title compound are obtained. Yield 94%, mp = 117-120 oC crystallized from ethyl acetate.

Characteristics of the product:

Mol. weight = 235.279 g/mole.

TLC ($CHCl_3$/metanol; 9.5/0.5 as the eluent) a sole spot Rf = 0.4.

$^1$H-RMN ($CDCl_3$, $\delta$ ppm.): 7.10-6.10 (3H,m,arom.,3CH), 2.53-1.93 (2H,wide absorption,2NH), 2.97 (8H,pseudo-singlet, $2CH_2CH_2$), 2.50-1.93 (1H,m,CH), 0.80-0.13 (4H,m, $CH_2CH_2$).

$^1$H-RMN (300 MHz,$CDCl_3$, $\delta$ ppm.): 6.8777 (1H,dd,$j_{H\text{-}F(orto)}$ = 11.64 Hz,$j_{H\text{-}H}$=8.725), 6.3521 (1H,d,$j_{H\text{-}F(meta)}$-=5.19 Hz), 6.3446 (1H,dd,$j_{H\text{-}F(meta)}$=7.02,$j_{H\text{-}H}$=8.725), 3.069-3.013 (8H,pseudo-singlet,$2CH_2CH_2$), 2.417-

2.294 ([1H,m,CH],[2H, wide absorption,2NH]), 0.739-0.680 (2H,m,$CH_2$), 0.518-0.469 (2H,m,CH).
IR ($CH_2Cl_2$, $cm^{-1}$): 3400 (N-H), 2810 (C-H).
Following the reaction:

By TLC ($CHCl_3$/methanol 9.5/0.5 as the eluent); the product obtained possesses a lower Rf (Rf = 0.4) than that of the starting material (Rf = 0.9).

Example 12

N-Cyclopropyl-3-chloro-4-fluoro-6-nitroacetanilide

23.06 g (10.0 cmole) of N-cyclopropyl-3-chloro-4-fluoro-6-nitroaniline are gradually added to 100 mL of acetic anhydride, endothermicity and solubilization being observed. 40 drops of conc. sulfuric acid (98 by weight) are added to the resulting orange suspension, a slight evolution of heat being observed. The mass is shaken at 40-50 oC for 120 min. and next at 80 oC, a reddish solution being obtained which is kept for 45 min at this temperature. It is cooled down to 0-5 oC, and 66 mL of methanol are slowly added (60 min.) keeping the temperature below 20 oC. This temperature is maintained during 30 min. while stirring. The mass is cooled down to 0-5 oC and 750 mL of a saturated aqueous solution of sodium bicarbonate is gradually added. It is extracted twice with 100 mL of dichloromethane, the organic layer is dried over anh. sodium sulfate and it is evaporated under reduced pressure. By filtration, a dark-brown product is obtained which is washed with 20 mL of n-hexane yielding 20.90 g (76.70%) of a light-colored crystalline product consisting in the title compound which is pure enough to be used in the next stage.

Characteristics of the product:
Mol. weight = 272.660 g/mole.
mp (crude) = 100-102 °C.
mp = 105-106 °C (recrystallized from a $CH_2Cl_2$/n-hexane mixture, white crystals). TLC (ethyl acetate/n-hexane; 1/1 as the eluent) a sole spot Rf = 0.5-0.6.
$^1$H-RMN ($CDCl_3$, δ ppm.): 7.80 (1H,arom,d,$j_{H-F(ortho)}$=10 Hz), 7.37 (1H,arom,d,$j_{H-F(meta)}$=6 Hz), 3.43-2.87 (1H,m,CH), 2.30 (3H,s,$CH_3$), 1.27-0.43 (4H,m,$CH_2CH_2$). $^1$H-RMN (300 MHz, $CDCl_3$, δ ppm. ): 7.784-(1H,d,arom.,$j_{H-F(ortho)}$ = 8.06 Hz), 7.359 (1H,d,arom.,$j_{H-F(meta)}$=6.60 Hz), 3.161-3.110 (1H,m,CH), 2.384 (s-[3H,$CH_3$],[27%3H,s,$CH_3$]), 1.647 (73%3H,s,$CH_3$ from isomer), 1.647-0.680 (4H, banda ancha, $CH_2CH_2$).
IR(KBr, $cm^{-1}$): 1670 (C=O), 1538 (N-O ass. str.).
Microanalysis:
Calculated: C, 48.46, H, 3.70, N, 10.27%.
Found: C, 48.16, H, 3.65, N, 10.27%.
Following the reaction:

By TLC (ethyl acetate/n-hexane 1/1 as the eluent); the reaction product displays an Rf = 0.6, and the starting material, an Rf = 0.9.

Following the same procedure, if instead of the nitro compound N-cyclopropyl-2-nitro-4,6-difluoro-5-chloroaniline is used, the corresponding acetanilide is obtained in a similar yield.

Example 13

N-Cyclopropyl-6-amino-3-chloro-4-fluoroacetanilide

47.50 g (20.0 cmole) of Ni(II) chloride hexahydrate are added to a solution of 27.26 g (10.0 cmole) of N-cyclopropyl-3-chloro-4-fluoro-6-nitroacetanilide in 375 mL of methanol, a green suspension being obtained which is cooled down from -10 to 0 oC. To the resulting suspension 15.10 g (40.0 cmole) of sodium borohydride in small portions are added, avoiding the temperature after each addition to rise above 0-5 oC. The reaction time is about 50 min., darkening of the suspension being observed. The reaction is strongly exothermical, with gaseous evolvement. After the addition, the temperature is kept at 0-5 oC for 15 min., and the resulting solution containing a black residue is filtered through celite and washed with 50 mL of methanol.

The filtrates are evaporated under reduced pressure and an oil is obtained which is dissolved in 100 mL of dichloromethane and washed with 50 mL of water; the organic layer is dried over anh. sodium sulfate and evaporated under reduced pressure. 20.63 g (85.02%) of an amorphous white solid of the title compound

are obtained which are provided with sufficient purity to be used in the next stage.

Characteristics of the product:
Mol. weight = 242.671 g/mole.
mp (crude) = 129-131 °C.
mp (recryst.) = 135-136 °C (from ethanol, a white crystals are obtained).
TLC (ethyl acetate/n-hexano; 1/1 as the eluent), a sole spot Rf = 0.2).
$^{1}$H-RMN ($CDCl_3$, δ ppm. ): 6.93 (1H,d,arom.,$j_{H-F(meta)}$ = 6 Hz), 6.57 (1H,arom. d,$j_{H-F(ortho)}$ = 12 Hz), 4.37-3.57 (2H,wide absorption,N-H), 2.33 (32%3H,wide absorption,$CH_3$ from an isomer), 1.83 (68%3H,wide absorption,$CH_3$ from isomer), 1.10-0.17 (4H,m,$CH_2CH_2$).
$^{1}$H-RMN (300 MHz, $CDCl_3$, δ ppm.): 6.911 (1H,d,arom.,$j_{H-F(meta)}$ = 7.52 Hz), 5.66 (1H,d,arom.,$j_{H-F(ortho)}$- = 10.25 Hz), 3.987-3.850 (2H,wide absorption,NH), 3.335-3.288 (1H,m,CH), 1.859(3H,s,$CH_3$) 0.769-0.387 (4H,m,$CH_2CH_2$).
IR (KBr, $cm^{-1}$): 1410, 3301 ($NH_2$), 1630 (C=O).
Microanalysis:
Calculated: C, 54.44, H,4.98, N, 11.54%.
Found: C, 54.35, H, 4.84, N, 11 .38%.
Following the reaction:

By TLC (ethyl acetate/n-hexane 1/1 as the eluent), the reaction product has an Rf = 0.2, being 0.6 that of the starting material.

Following the same procedure N-cyclopropyl-2-amino-4,6-difluoro-5-chloroacetanilide and N-cyclopropyl-2-amino-4-fluoro-5,6-dichloroaniline are obtained.

Example 14

N-Cyclopropyl-3-chloro-4-fluoroacetanilide

A solution of N-cyclopropyl-6-amino-3-chloro-4-fluoroacetanilide (24.3 g, 0.10 mole) in methanol (sufficient amount) is prepared and then added into another solution, cooled down to 0-5 oC of 50% hypophosphorous acid (164.0 mL, 1.50 mole) containing sulfuric acid (0.20 mole) and copper sulfate (1.64 g, 0.01 mole). Maintaining the temperature (0-5 oC) and with good stirring another solution of sodium nitrite (7.2 g, 0.104 mole) in water (50 mL) is added. The mass is stirred at 0-5 oC until the gaseous evolvement is over. It results in an intense blue solution. The mixture is then extracted with dichloromethane (2 x 175 mL), washed with a small amount of ammonia solution, the color changing to light brown. It is dried and its solvent is evaporated under reduced pressure. The resulting red oil begins to crystallize at room temperature. Shaking with a minimal amount of n-hexane, it crystallizes, and it is filtered, washed and dried, yielding 20.55 g of the title compound. Yield: 90.0%; mp = 50-52 oC (from n-hexane).

Characteristics of the product:
Mol. weight = 226.640 g/mole.
mp = an oil.
TLC (ethyl acetate/n-hexano; 1/1 as the eluent) a sole spot Rf = 0.5.
$^{1}$H-RMN ($CDCl_3$, δ ppm.): 7.50-6.80 (2H,arom.,m), 7.07 (1H,arom.,d.$j_{H-F(meta)}$ = 8 Hz), 3.33-2.83 (1H,m,CH), 2.07 (3H,s,$CH_3$), 1.07-0.23 (4H,m,$CH_2CH_2$).
IR (KBr, $cm^{-1}$): 1670 (C=O).
Following the reaction:

By TLC (n-hexane as the eluent), the product obtained has an Rf = 0.0 while that of the starting material is Rf = 0.4. Following the same method, starting from N-cyclopropyl-6-amino-2,3-dichloro-4-fluoroacetanilide, N-cyclopropyl-6-amino-3-chloro-2,4-difluoroacetanilide, N-cyclopropyl-6-amino-3-chloro-2,4-difluoro-5-methylacetanilide and N-cyclopropyl-6-amino-2,3-dichloro-4-fluoroacetanilide the corresponding deaminated products in similar yields result.

Example 15

N-Cyclopropyl-3-chloro-4-fluoroaniline

To a solution of NaOH (12.0 g, 0.30 mole) in methanol/water (100 mL/25 mL) 22.8 g (0.10 mole) of N-cyclopropyl-3-chloro-4-fluoroacetanilide are added. The resulting solution is heated to reflux (74 oC) for 2 h. During the heating darkening of the reaction mixture is observed. The resulting solution is cooled down to room temperature, diluted with 100 mL of water, and extracted twice with 100 mL of dichloromethane. The organic layer is washed with 0.1 N hydrochloric acid (100 mL), dried over anh. sodium sulfate and evaporated under reduced pressure yielding 15.7 g (84.8%) of an orange oil of the title compound.

Characteristics of the product:
Mol. weight = 184.602 g/mole.
mp = an oil.
TLC ($CH_2Cl_2$/methanol; 2/8 as the eluent), a sole spot Rf = 0.8.
$^1$H-RMN ($CDCl_3$, $\delta$ ppm. ): 7.17-6.50 (2H,arom.,m), 6.90 (1H,arom.,d,$j_{H-F(meta)}$ = 8Hz), 6.50-6.17 (1H,wide absorption, N-H), 2.63-2.17 (1H,m,CH), 0.83-0.47 (4H,m,$CH_2CH_2$).
IR (KBr, $cm^{-1}$): 3410, 3080, 3000, 2955, 1610, 1512, 1365, 1255, 1228.
Following the reaction:

By TLC (ethyl acetate/n-hexane 1/1 as the eluent); the product has an Rf = 0 while that of the starting material is Rf = 0.5.

For the 2-fluoro, 2-chloro and 5-methyl derivatives, the hydrolysis is effected by shaking during 120 min., at the temperature of 40-50 oC, completing it next as in the preceding procedure (refluxing for 30 min.)

## Claims

1. A procedure for the preparation of N-cyclopropyl-4-fluoroanilines of formula I

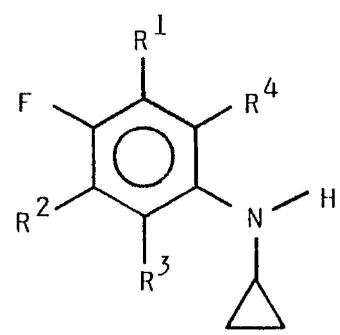

I

where $R^1$ represents a hydrogen atom, a chlorine or a methyl group; $R^2$, a fluorine atom, chlorine, bromine, a methyl group, a piperazinyl, pyrrolyl, pyrrolidinyl or 2-oxo-1,3-oxazolidinyl; $R^3$, a hydrogen atom, chlorine, fluorine, hydroxyl or trifluoromethyl; and $R^4$, a hydrogen atom, a nitro group, hydroxyl or amino, characterized because it incorporates:

(i) a nitrobenzene of formula II,

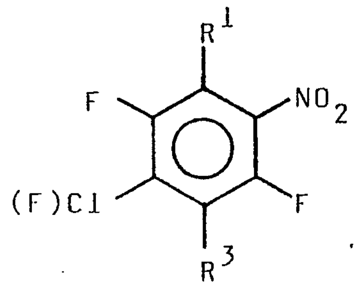

II

$R^1$ and $R^2$ having the meaning given before, which is made to react selectively first with

cyclopropylamine at room temperature, and second, with a piperazine, pyrrolidine, pyrroline, imidazoline or 2-oxo-1,3-oxazolidine at the temperature from 50 oC to 70 oC to obtain a nitrobenzene of formula III,

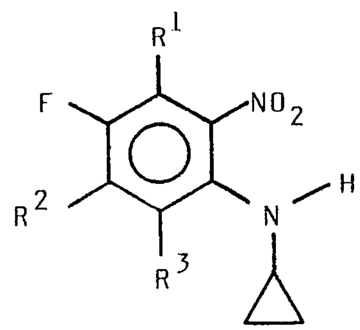

III

$R^1$, $R^2$ and $R^3$ having the meaning given before for compound of formula I, except $R^2$ a chlorine or fluorine atom;

(ii) acetylate compound of formula III, being effected with acetic anhydride or acetyl chloride, to obtain a nitrobenzene of formula IV,

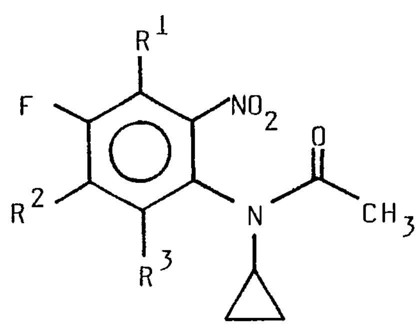

IV

$R^1$, $R^2$ and $R^3$ having the meaning given, and $R^2$ being a group provided with a NH, and $R^3$, a hydroxyl, resulting the compound N-acetyl and O-acetyl;

(iii) reduce the compound of formula IV in methanol, ethanol or isopropanol solution under the pressure of 1 to 3 bar and the temperature of 0 oC to 25 oC, with 5 to 10% palladium/carbon, with sodium borohydride-nickel chloride, or selectively with sodium borohydride-trimethylchlorosilane, to obtain an aniline of formula V,

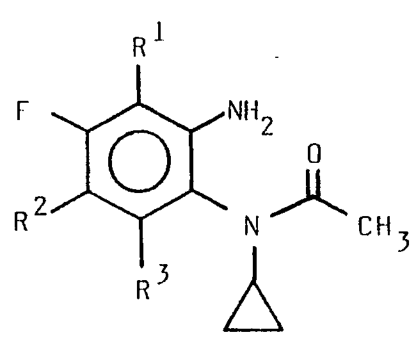

V

where $R^1$, $R^2$ and $R^3$ have the meaning given above, which by acidic hydrolysis yields a compound

of formula I, being $R^4$ an amino group;
(iv) reductive diazotation of compound of formula V with an alkaline nitrite and a reductant chosen among hypophosphorous acid, glucose, formaldehyde, formic acid or a combination of them, in the presence of copper, to obtain an aniline of formula VI,

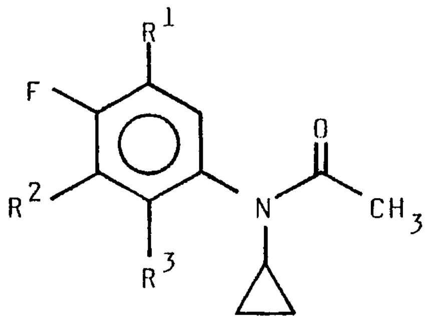

VI

where $R^1$, $R^2$ and $R^3$ have the meaning given above, which by hydrolysis yields a compound of formula I, being $R^4$ a hydrogen atom; and
(v) isolation of the compounds as bases or salts.

2. A procedure according to the first claim, for the preparation of compounds Ia and Ib,

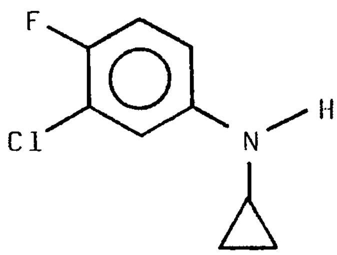

Ia

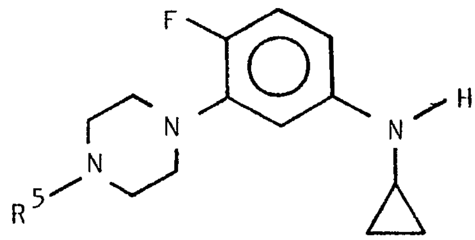

Ib

being $R^5$ a hydrogen atom, methyl, ethyl or cycloprcpyl, characterized because it includes:
(i) the selective reaction of a nitrobenzene of formula II, being $R^1$ and $R^3$ hydrogen atoms, first with cyclopropylamine to obtain the compound of formula IIIa,

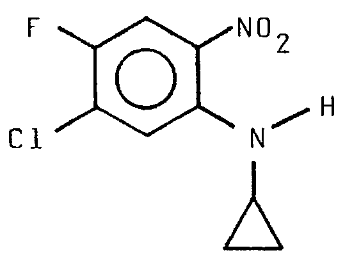

IIIa

and next with a piperazine to obtain a compound of formula IIIb,

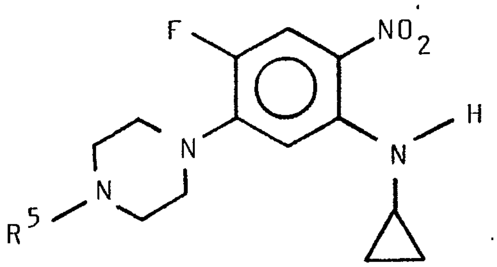

IIIb

$R^5$ meaning as before,

(ii) acetylate compounds IIIa and IIIb to obtain the N-acetylated compounds IVa and IVb,

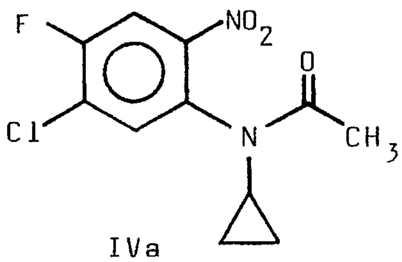

IVa

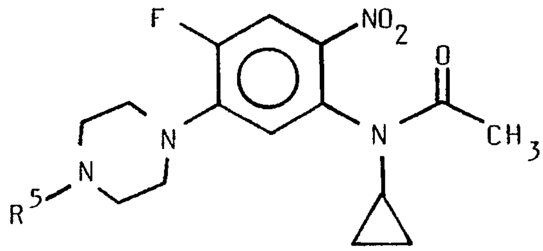

IVb

$R^5$ meaning as before,

(iii) reduce compounds IVa and IVb in ethanol solution at a temperature from -5 o to 10 oC to obtain an aniline of formula Va and Vb,

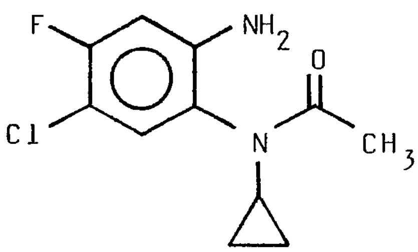

Va

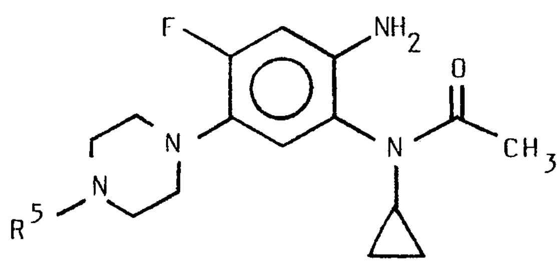

Vb

the meaning of $R^5$ being that given before.

(iv) reductive diazotation of Va and Vb with sodium nitrite, and its reaction with hypophosphorous acid to obtain compounds VIa and VIb,

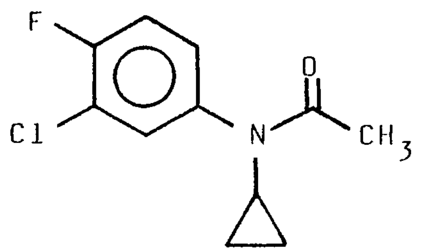

VIa

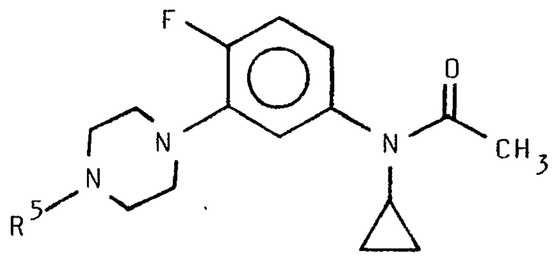

VIb

$R^5$ meaning as before, which by hydrolysis yield the fluoroanilines of formula Ia and Ib.

3. A procedure, according to the first claim, for the preparation of the following anilines:
   N-cyclopropyl-3-chloro-4-fluoro-5-methylaniline,
   N-cyclopropyl-2,3-dichloro-4-fluoroaniline,
   N-cyclopropyl-2,3,4-trifluoroaniline,
   N-cyclopropyl-3-chloro-2,4-difluoroaniline,
   N-cyclopropyl-3-chloro-4-fluoro-2-hydroxyaniline,
   N-cyclopropyl-2,4-difluor-3-chloro-5-methylaniline, and its salts.

4. A procedure, according to the first claim, for the preparation of the following nitroanilines:
   N-cyclopropyl-3-chloro-4-fluoro-5-methyl-6-nitroaniline,
   N-cyclopropyl-2,3-dichloro-4-fluoro-6-nitroaniline,
   N-cyclopropyl-2,3,4-trifluoro-6-nitroaniline,
   N-cyclopropyl-3-chloro-4-fluoro-2-hydroxy-6-nitroaniline,
   N-cyclopropyl-2,4-difluoro-3-chloro-6-nitroaniline,
   N-cyclopropyl-2,4-difluoro-3-chloro-5-methyl-6-nitroaniline, and its salts.

5. A procedure, according to the first claim, for the preparation of the following fluoroanilines:
   N-cyclopropyl-3-(4-ethyl-1-piperacinyl )-4-fluoroaniline,
   N-cyclopropyl-3-(3-methyl-1-piperacinyl )-4-fluoroaniline,
   N-cyclopropyl-3-(3-hydroxy-1-pyrrolidinyl )-4-fluoroaniline,
   N-cyclopropyl-3-(3-hydroxy-1-pyrrolidinyl)-2-chloro-4-fluoroaniline,
   N-cyclopropyl-3-[3-(1-aminoethyl)-1-pyrrolidinyl]-2-trifluoromethyl-4-fluoroaniline,
   N-cyclopropyl-3-(4-cyclopropyl-1-piperacinyl )-4-fluoroaniline,
   N-cyclopropyl-2-chloro-3-(1-piperacinyl)-5-methyl-4-fluoroaniline,
   N-cyclopropyl-2-chloro-3-(1-piperacinyl )-4-fluoroaniline,
   N-cyclopropyl-2-fluoro-3-(1-piperacinyl )-5-methyl-4-fluoroaniline,
   N-cyclopropyl-3-(3-amino-1-pyrrolidinyl )-2-trifluoromethyl-4-fluoroaniline,
   N-cyclopropyl-3-[3-(2-oxo-1,3-oxazolidinyl )]-4-fluoroaniline,
   N-cyclopropyl-2-fluoro-3-[3(2-oxo-1,3-oxazolidinyl)]-4-fluoroaniline,
   N-cyclopropyl-2-chloro-3-[3(2-oxo-1,3-oxazolidinyl)]-4-fluoroaniline, and its salt and N-acetyl derivatives.

6. A procedure, according to the first claim, characterized because for $R^2$ are preferentially chosen piperazine, 3-methylpiperazine, 4-acetyl-3-methylpiperazine, 4-ethyl-piperazine, 4-methylpiperazine, 4-cyclopropilpiperazine, pyrrol, 3-aminoethylpyrrolidine, 3-aminomethylpyrrolidine, 3-aminopyrrolidine, 3-acetylaminopyrrolidine, 3-hydroxipyrrolidine, 3-hydroximethylpyrrolidine, 3-cyclopropilaminopyrrolidine and 2-oxo-1,3-oxazolidinone.

7. A procedure, according to the first claim, characterized because the selective reaction of a nitro compound of formula II with cyclopropylamine is performed in a solvent chosen among the dimethylsulfoxide, dimethylformamide and their mixtures, at a temperature from 20 ºC to 50 ºC.

8. A procedure, according to the first claim, characterized because the reduction of a compound of

formula IV is performed by means of sodium borohydride in the presence of three equivalents of a nickel compound and at a temperature from -5 ºC to 10 ºC.

**9.** A procedure, according to the first claim, characterized because the reductive diazotation reaction of a compound of formula V is effected with an alkaline nitrite and hypophosphorous acid, at room temperature and in the presence of a catalyst chosen among the copper compounds, nitrate, sulfate, oxide, chloride, phosphate, hypophosphite and phosphite.

European Patent Office

# EUROPEAN SEARCH REPORT

Application Number

EP 90500109.5

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP - A1 - 0 195 316 (KYORIN PHARMACEUTICAL) * Examples 7-10 * -- | 1 | C 07 C 211/52<br>C 07 C 209/00<br>C 07 D 295/125 |
| X | EP - A1 - 0 183 129 (KYORIN PHARMACEUTICAL) * Reference examples 7-10 * -- | 1 | |
| X | PATENT ABSTRACTS OF JAPAN, unexamined applications, E field, vol. 10, no. 339, November 15, 1986 THE PATENT OFFICE JAPANESE GOVERNMENT page 51 C 385 * Kokai-No. 61-143 339 (KYORIN PHARMACEUTICAL) * -- | 1 | |
| A | DE - A1 - 3 705 621 (OTSUKA PHARMACEUTICAL) * Page 55, reference example 31 * ---- | 1,7 | |

TECHNICAL FIELDS SEARCHED (Int. Cl.5)

C 07 C 211/00
C 07 C 209/00
C 07 D 295/00
C 07 D 401/00

The present search report has been drawn up for all claims

| Place of search | Date of completion of the search | Examiner |
|---|---|---|
| VIENNA | 25-03-1991 | KÖRBER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document